# EUROPEAN PATENT SPECIFICATION

(11) **EP 2 303 141 B1**
(45) Date of publication and mention of the grant of the patent: **26.09.2018**
(21) Application number: 09770391.2
(22) Date of filing: 24.06.2009
(51) Int. Cl.: A61B 17/00

(54) **TOOL FOR MINIMALLY INVASIVE SURGERY**
INSTRUMENT FÜR DIE MINIMALINVASIVE CHIRURGIE
INSTRUMENT DE CHIRURGIE MINI-INVASIVE

(30) Priority: 27.06.2008 KR 20080061894
(43) Date of publication of application: 06.04.2011
(73) Proprietor: Jeong, Chang Wook, Seoul 135-796 (KR)
(72) Inventor: Jeong, Chang Wook, Seoul 135-796 (KR)
(74) Representative: Zardi, Marco
(86) International application number: PCT/KR2009/003417
(87) International publication number: WO 2009/157719

(56) References cited:
- EP-A1- 1 695 669
- EP-A1- 1 854 418
- WO-A2-03/001986
- JP-A- 2006 061 364
- US-A- 1 917 929
- US-A1- 2002 111 621
- US-A1- 2004 167 515
- US-A1- 2004 199 147
- US-A1- 2007 208 375
- US-B1- 6 394 998
- US-B1- 6 902 560
- US-B1- 6 902 560

## Description

### Technical Field

The present invention relates to an easy-to-control tool for minimally invasive surgery, and more specifically, to a tool, which includes an adjustment handle connected to one end of a predetermined shaft and an end effector that is connected to the other end of the shaft and controllable merely through the actuation of the adjustment handle, so as to perform minimally invasive surgery.

### Background Art

Minimally invasive surgery is a surgical approach that involves use of instruments inserted through several tiny incision openings to perform a surgery causing minimal tissue trauma.

This minimally invasive surgery relatively reduces changes in metabolism of the patient in the period of post-surgical care, so it is beneficial to rapid recovery of the patient. Therefore, using such minimally invasive surgery shortens length of a hospital stay of the patient after the surgery and allows patients to return to normal physical activities more quickly. In addition, minimally invasive surgery causes less pain and reduces scar to patients after surgery.

The most general form of the minimally invasive surgery is endoscopy. Among them, a laparoscopy that involves minimally-invasive inspection and operation inside abdominal cavity is known as the most general form of endoscopy. To operate the standard laparoscopic surgery, an abdomen of the patient is insufflated with gas, and small incisions (about 1/2 inch or less) are formed for use as an entrance of a tool for the laparoscopic surgery, through which a trocar is inserted. In general, laparoscopic surgical tools include a laparoscope (for observation of a surgical site) and other working tools. Here, the working tools are similar in structure to the conventional tools used for small incision surgery, except that the end effector or working end of each tool is separated from its handle by an elongated shaft. For instance, working tools may include a clamp, a grasper, scissors, a stapler, needle holder, and so forth. To perform the surgery, a user, such as a surgeon, puts the working tool into a surgical site through the trocar, and manipulates it from the outside of abdominal cavity. Then, the surgeon monitors the procedure of the surgery through a monitor that displays the image of the surgical site that is taken by the laparoscope. The endoscopic approach similar to this is broadly used in retroperitoneoscopy, pelviscopy, arthroscopy, cisternoscopy, sinuscopy, hysteroscopy, nephroscopy, cystoscopy, urethroscopy, pyeloscopy, and so on.

US 2004/167515 A1, WO 03/001986A2, US 6 394 998 B1, US 1 917 929 A, US 6 902 560 B1, EP 1 854 418 A1, EP 1 695 669 A1, and US 2004/199147 A1 disclose relevant prior arts, which disclose a portion of features recited in the preamble of claim 1.

US2007208375 discloses a surgical instrument according to the preamble of claim 1.

### Technical Problem

Although this minimally invasive surgery has a number of advantages, it has shortcomings in the difficulty of approaching the conventional minimally invasive surgical tools to a surgical site and the inconvenient or complicate manipulation of such tools because of an end effector connected to a rigid and long shaft. Particularly, since the traditional end effector has no bending portion like a joint, it is difficult to perform a dexterous handling required for surgery. These shortcomings are the main impediment to the wide expansion of minimally invasive surgery.

To overcome these shortcomings of the traditional minimally invasive surgery, recently, a robotic assisted platform called the da Vinci®surgical system has been developed by Intuitive Surgical, Inc. The robotic assisted surgical system currently being commercialized mainly uses a master-slave type robot, which is constituted by an operating console where an operator performs an operation, a robotic cart where a robot performs an operation, and an endoscopic stack being connected thereto. An endoscopic stack in the robotic surgical system has a joint that can move in a pitch direction and a yaw direction, and thus can transfer hand motions of the operator almost exactly. Also, the robotic surgical system has a function of tremor reduction or a function of motion scaling to differentiate robot motion from hand motion in terms of scale, and can secure a three dimensional vision.

However, this robotic surgical system is very expensive equipment, and moreover, it costs a tremendous amount of money to install and maintain after installation. This equipment is also bulky and very heavy (even the robotic cart alone is about 2m tall and as heavy as 544kg). Needless to say, it is difficult to move the equipment around, so the surgery has to be performed only in a place where the system is already installed. Besides, in case of using the robotic system, surgeons feel lack of tactile sense, as compared with using the traditional tools for laparoscopic surgery.

### Technical Solution

The invention is disclosed in the present set of claims. The present invention is directed to solve all of the problems discussed above.

It is, therefore, an object of the present invention to provide a tool for minimally invasive surgery with an end effector that operates in correspondence to motions in pitch/yaw directions and/or opening and closing operations.

Another object of the present invention is to provide a tool for minimally invasive surgery, which a user can freely actuate without the help of a special drive element.

A still further object of the present invention is to provide a tool for minimally invasive surgery, which features small volume, lightweight, and convenient movability.

### Advantageous Effects

As discussed earlier, the tool for minimally invasive surgery in accordance with the present invention is provided with an end effector which features high-degree-of-freedom motion corresponding to the user's manual control over an adjustment handle.

In addition, the tool for minimally invasive surgery in accordance with the present invention is configured for any user to operate with easiness.

Moreover, the tool for minimally invasive surgery in accordance with the present invention can be manufactured and supplied at low costs, and has small volume and lightweight, making easier to supply.

### Brief Description of Drawings

The above and other objects and features of the present invention will become apparent from the following description of preferred embodiments, given in conjunction with the accompanying drawings, in which:
Fig. 1 is a perspective view showing the outer appearance of a tool for minimally invasive surgery in accordance with a first embodiment of the present invention;
Figs. 2 and 3 are detailed views showing the configuration of the tool for minimally invasive surgery in accordance with the first embodiment of the present invention;
Fig. 4 is an exploded perspective view showing a connection status between a shaft and an adjustment handle in accordance with the first embodiment of the present invention;
Fig. 5 is an exploded perspective view showing a connection status between a shaft and an end effector in accordance with the first embodiment of the present invention;
Figs. 6, 7 and 8 show an installation status of pitch axis actuating cables and yaw axis actuating cables on the side of the adjustment handle in accordance with the first embodiment of the present invention;
Figs. 9, 10 and 11 show an installation status of pitch axis actuating cables and yaw axis actuating cables on the side of the end effector in accordance with the first embodiment of the present invention;
Figs. 12, 13 and 14 show a usage example of the tool for minimally invasive surgery in accordance with the first embodiment of the present invention;
Figs. 15, 16 and 17 are conceptual diagrams describing the principle of transfer of motions of first and second connection pulleys in accordance with the first embodiment of the present invention;
Figs. 18, 19, 20, 21 and 22 are diagrams showing the configuration of a tool for minimally invasive surgery in accordance with a second aspect of the present disclosure, which is not part of the invention;
Figs. 23, 24, 25 and 26 are diagrams showing the configuration of a tool for minimally invasive surgery in accordance with a third embodiment of the present invention;

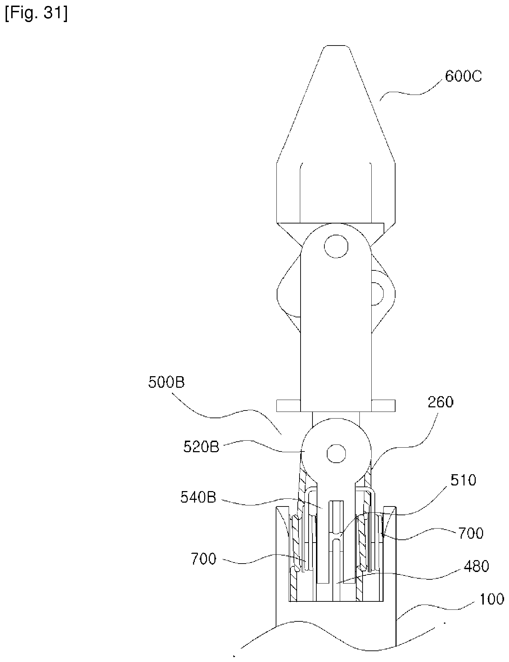

Figs. 27, 28, 29, 30, 31, 32 and 33 are diagrams showing the configuration of a tool for minimally invasive surgery in accordance with a fourth embodiment of the present invention; and
Fig. 34 is a diagram showing a modified configuration of the tool for minimally invasive surgery in accordance with the fourth embodiment of the present invention.

### Best Mode for Carrying out the Invention

In accordance with an aspect of the present invention, there is provided a tool for minimally invasive surgery comprising, an elongated shaft having a predetermined length, an adjustment handle manually controllable by a user, a first pitch axis part and a first yaw axis part positioned around one end of the elongated shaft for transferring motions in pitch and yaw directions following the operation of the adjustment handle, a second pitch axis part and a second yaw axis part positioned around the other end of the elongated shaft for operating corresponding to the operations from the first pitch axis part and the first yaw axis part, respectively, and an end effector controllable by the second pitch axis part and the second yaw axis part, wherein the first pitch axis part drives the second pitch axis part by at least one pitch axis actuating cable, and the first yaw axis part drives the second yaw axis part by at least one yaw axis actuating cable.

In accordance with another aspect of the present invention, there is provided a tool for minimally invasive surgery comprising, an elongated shaft having a predetermined length, an adjustment handle manually controllable by a user, a first pitch axis part and a second yaw axis part positioned around one end of the elongated shaft for transferring motions in pitch and yaw directions following the operation of the adjustment handle, a second pitch axis part and a second yaw axis part positioned around the other end of the elongated shaft for operating corresponding to the operations from the first pitch axis part and the first yaw axis part, respectively, and an end effector controllable by the second pitch axis part and the second yaw axis part, wherein the first pitch axis part and the first yaw axis part drive the second pitch axis part and the second yaw axis part by at least one actuating cable, respectively.

In accordance with still another aspect of the present invention, there is provided a tool for minimally invasive surgery comprising, an elongated shaft having a predetermined length, an elongated shaft having a predetermined length, an adjustment handle manually controllable by a user, a first pitch axis part and a second yaw axis part positioned around one end of the elongated shaft for transferring motions in pitch and yaw directions following the operation of the adjustment handle, a second pitch axis part and a second yaw axis part positioned around the other end of the elongated shaft for operating corresponding to the operations from the first pitch axis part and the first yaw axis part, respectively, and an end effector controllable and configured to be opened and closed by the second pitch axis part and the second yaw axis part, wherein the first pitch axis part and the first yaw axis part drive the second pitch axis part and the second yaw axis part by at least one first cable and at least one second cable, and wherein the end effector is configured to open or close according to the difference in the displacement amount of the at least one first cable and the at least one second cable.

### Mode for the Invention

In the following detailed description, reference is made to the accompanying drawings that show, by way of illustration, specific embodiments in which the invention may be practiced. These embodiments are described in sufficient detail to enable those skilled in the art to practice the invention. It is to be understood that the various embodiments of the invention, although different, are not necessarily mutually exclusive. The following detailed description is, therefore, not to be taken in a limiting sense, and the scope of the present invention is defined only by the appended claims that should be appropriately interpreted along with the full range of equivalents to which the claims are entitled.

Also, it is to be understood that the indicative terms of particular operational directions included in the names of particular elements of the present invention do not necessarily concern a corresponding direction or an operation in that direction only, and contribution of each element to the present invention should be understood in terms of a specific operation of the corresponding element.

Hereinafter, preferred embodiments of the present invention will be explained in detail with reference to the accompanying drawing.

### Embodiment I

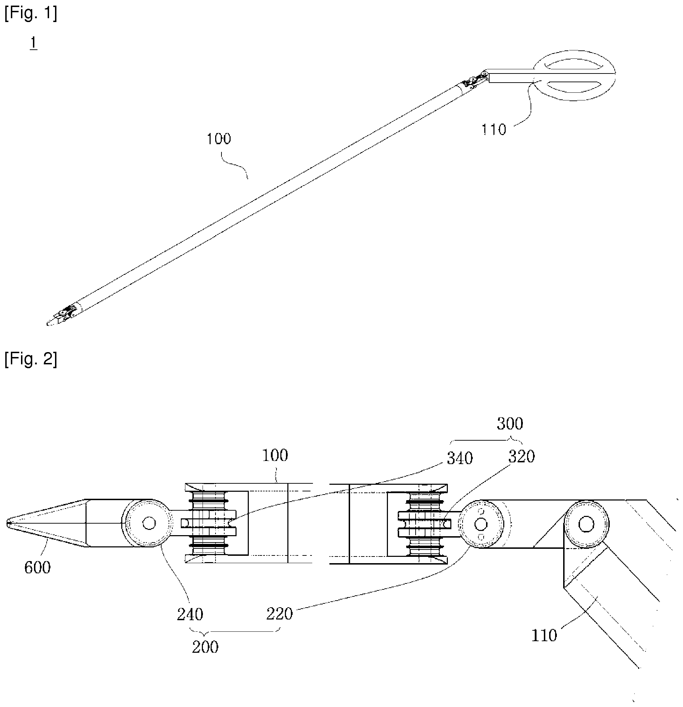

Fig. 1 is a perspective view showing the outer appearance of a tool 1 for minimally invasive surgery, in accordance with a first embodiment of the present invention. Referring to Fig. 1, the tool 1 for minimally invasive surgery includes an elongated shaft 100 of a predetermined length, which has one or plural spaces inside (e.g., pipe-shape, lotus-shaped, or spiral-shaped space), and an adjustment handle 110.

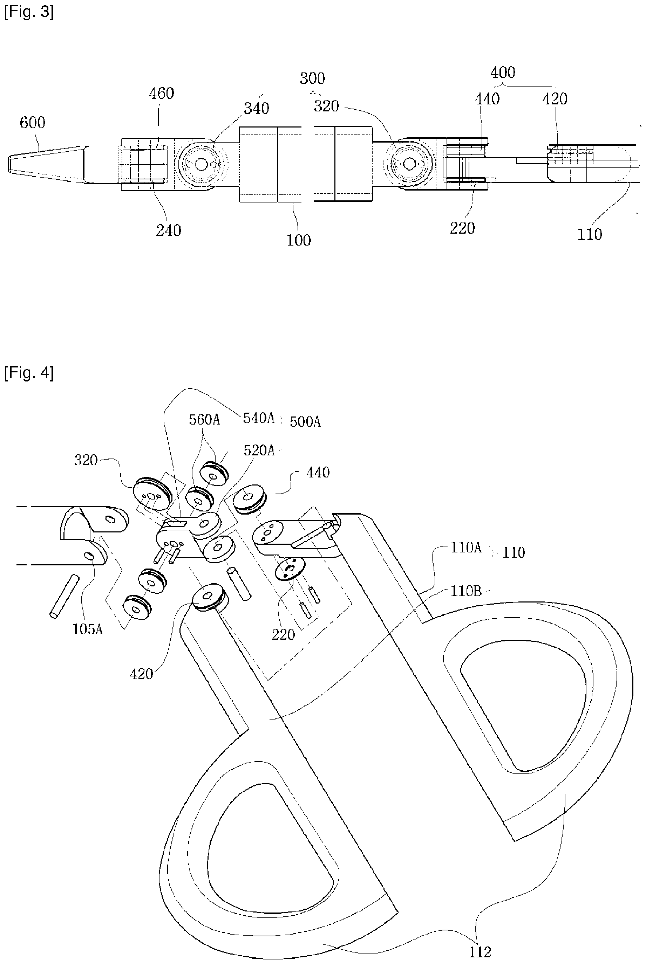

Fig. 2 is a side view showing a detailed configuration of main elements such as a pitch axis part 200 and a yaw axis part 300 in accordance with a first embodiment of the present invention, and Fig. 3 is a plan view showing a configuration of main elements as a yaw axis part 300 in accordance with the first embodiment of the present invention.

As shown in Figs. 2 and 3, a tool 1 for minimally invasive surgery in accordance with the first embodiment of the present invention includes an elongated shaft 100, and an adjustment handle 110 and an end effector 600 disposed on both ends of the elongated shaft 100. Here, it is configured in a manner that the operation of the adjustment handle 110 is transferred to the end effector 600 through a pitch axis part 200, a yaw axis part 300, and an opening and closing part 400. The end effector 600 connected to the elongated shaft 100 in accordance with the first embodiment of the present invention operates corresponding to the opening and closing operation of the adjustment handle 110, and is used as a tool for the surgery inside the body, such as, a clamp, a gasper, scissors, a stapler, a needle holder, etc.

If necessary, the end effector 600 in accordance with the present invention may be any element which does not necessarily need to be opened or closed, like a hook electrode. An embodiment of the end effector that does not require the opening and closing operation will be described later.

More specifically, referring to the drawings, the pitch axis part 200 includes a first pitch axis cable pulley 220, a second pitch axis cable pulley 240, and a pitch axis actuating cable; the yaw axis part 300 includes a first yaw axis cable pulley 320, a second yaw axis cable pulley 340, and a yaw axis actuating cable; and the opening and closing part 400 includes a first opening and closing cable pulley 420, a second opening and closing cable pulley 440, and a third opening and closing cable pulley 460.

Fig. 4 is an exploded perspective view showing a connection status between the elongated shaft 100 and the adjustment handle 110 in accordance with the first embodiment of the present invention.

As shown, first and second rods 110A and 110B are connected to each other by a rotation axis to configure the adjustment handle 110, and two enclosures 112 of a semi-circular shape are formed symmetrically to each other on one end of each of the first and the second rods 110A and 110B that are connected by the rotation axis. And the first opening and closing cable pulley 420 is installed on one side of the rotation axis of the adjustment handle 110, rotating following the opening and closing operation of the adjustment handle 110. The first opening and closing cable pulley 420 may be configured to have the same width as conventional opening and closing cables.

On the opposite, there is the first rod 110A being extended longitudinally, and the first pitch axis cable pulley 220 and the second opening and closing cable pulley 440 that constitute the pitch axis part 200 and the opening and closing part 400, respectively, are installed at both sides of the end of the elongated first rod 110A. Although the first pitch axis cable pulley 220 and the second opening and closing cable pulley 440 are installed on the same axis, the first pitch axis cable pulley 220 is securely held not to rotate, while the second opening and closing cable pulley 440 is installed to be able to rotate freely. In Fig. 4, the first pitch axis cable pulley 220 and the second opening and closing cable pulley 440 are the same in terms of diameter size, but different diameters may be utilized depending on what the user'S needs are.

Meanwhile, it is preferable that the first pitch axis cable pulley 220 is formed to be as wide as the pitch axis actuating cable, and the second opening and closing cable pulley 440 is formed to be three times wider than the opening and closing cable.

Also, interposed between the first pitch axis cable pulley 220 and the first yaw axis cable pulley 320 is a first axis connection part 500A in which a first pitch axis connection part 520A and a second yaw axis connection part 540A are disposed on their both ends, so that motions of the adjustment handle 110 in pitch and yaw directions can readily be transferred to the end effector.

To be more specific, the first axis connection part 500A includes a first pitch axis connection part 520A having a pair of circular shape plates spaced apart from each other by a predetermined distance, and a first yaw axis connection part 540A having a pair of circular shape plates spaced apart from each other by a predetermined distance. Here, as shown in Fig. 4, the first pitch axis connection part 520A and the first yaw axis connection part 540A are coupled orthogonally, so the first pitch axis cable pulley 220 which is disposed at one end of the adjustment handle 110 is rotatably settled on the inside of the first pitch axis connection part 520A and the first yaw axis cable pulley 320 is fixedly secured on the inside of the first yaw axis connection part 540A. The first yaw axis cable pulley 320 can have substantially the same width as the yaw axis actuating cable.

Also, each pair of first connection pulleys 560A is rotatably disposed on either outside of the first yaw axis connection part 540A. At this time, the first connection pulleys 560A and the first yaw axis cable pulley 320 are coaxially disposed and rotate independently of each other. Each of the first connection pulleys 560A is preferably formed to be about twice wider than the pitch axis actuating cable or the opening and closing cable.

Meanwhile, a pair of first connection parts 105A of predetermined length is protruded in parallel from one end of the elongated shaft 100 to which the adjustment handle 110 is connected. The first yaw axis cable pulley 320 that has been positioned at the first yaw axis connection part 540A is disposed between the first connection parts 105A to be rotatable about the central axis.

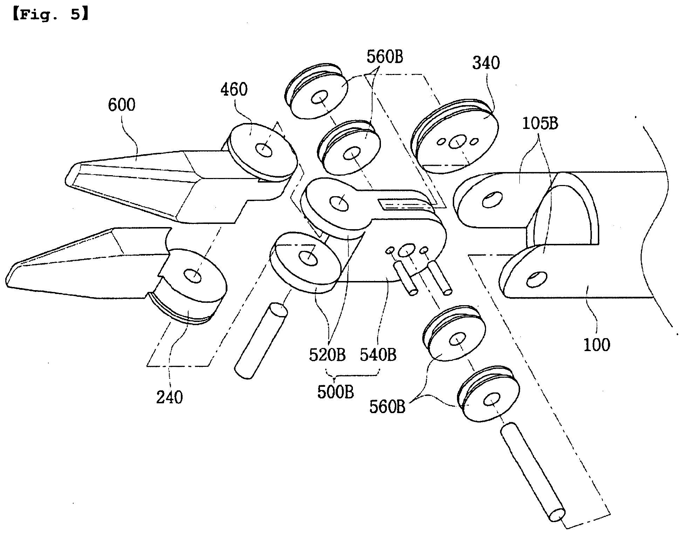

Fig. 5 is an exploded perspective view showing a connection status between the elongated shaft 100 and the end effector 600 in accordance with the first embodiment of the present invention. Referring to Fig. 5, on one end of the shaft 100, that is, on the end where the end effector 600 is disposed, the second pitch axis cable pulley 240 and the second yaw axis cable pulley 340 are connected by a pair of second connection parts 105B. Preferably, the second connection parts 105B may be configured in the same manner as the first connection parts 105A.

More detailed descriptions on the installation status of the second pitch axis cable pulley 240 and the second yaw axis cable pulley 340 will now be provided below, with reference to the drawing.

First, a pair of the second connection parts 105B is protruded in parallel from one end of the shaft 100 on which the end effector 600 is disposed, and a second axis connection part 500B that has a second pitch axis connection part 520B and a second yaw axis connection part 540B on both ends is connected to the second connection parts 105B. Since the second axis connection part 500B constituted by the second pitch axis connection part 520B and the second yaw axis connection part 540B is configured in the same manner as the first axis connection part 500A, more details thereof will be omitted here.

The second yaw axis cable pulley 340 is fixedly disposed between the second yaw axis connection parts 540B, and the central axis of the second yaw axis cable pulley 340 is coaxially formed with the central axis of the second yaw axis connection parts 540B. At this time, the second yaw axis cable pulley 340 has substantially the same width as the yaw axis actuating cable. If a user (e.g., surgeon) wants the operation amount of the adjustment handle 110 in the yaw direction to be equal to the displacement amount of the end effector 600 in the yaw direction, the second yaw axis cable pulley 340 can be formed to have the same diameter as the first yaw axis cable pulley 320. On the other hand, if the user wants the operation amount of the adjustment handle 110 in the yaw direction to be different from the displacement amount of the end effector 600 in the yaw direction, the aforementioned two elements may be formed to have different diameters from each other.

In addition, referring to Fig. 5, the end effector 600, which is constituted by two rods in the shape of an extended right triangle with a rotation axis connecting one end of each rod, is rotatably connected to the second pitch axis connection parts 520B of the second axis connection part 500B. Besides, the second pitch axis cable pulley 240 is fixedly disposed on one end of a connection axis which connects the end effector 600 with the second pitch axis connection parts 520B, such that the end effector 600 operates in the pitch direction following the rotation of the second pitch axis cable pulley 240. Meanwhile, a third opening and closing cable pulley 460 is disposed on the other side end of the connection axis, thereby making the end effector 600 opened or closed. In this manner, the second pitch axis cable pulley 240 and the third opening and closing cable pulley 460 operate independently of each other.

Meanwhile, although the second pitch axis cable pulley 240 and the third opening and closing cable pulley 460 are the same in terms of diameter size by way of example, they may also have different diameters depending on what the user's needs are. Also, the second pitch axis cable pulley 240 and the third opening and closing cable pulley 460 have substantially the same width as each cable.

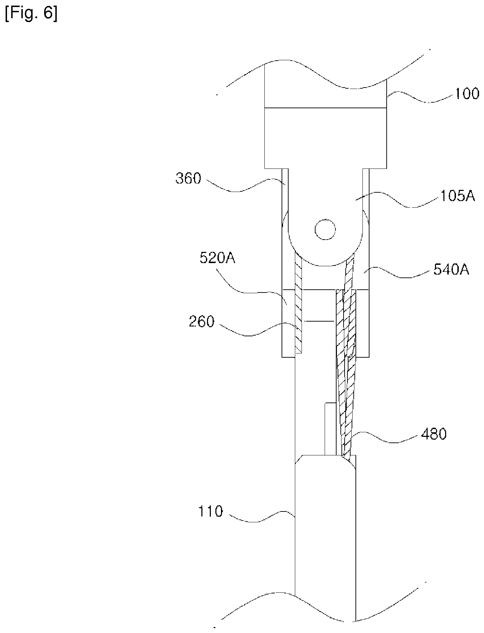

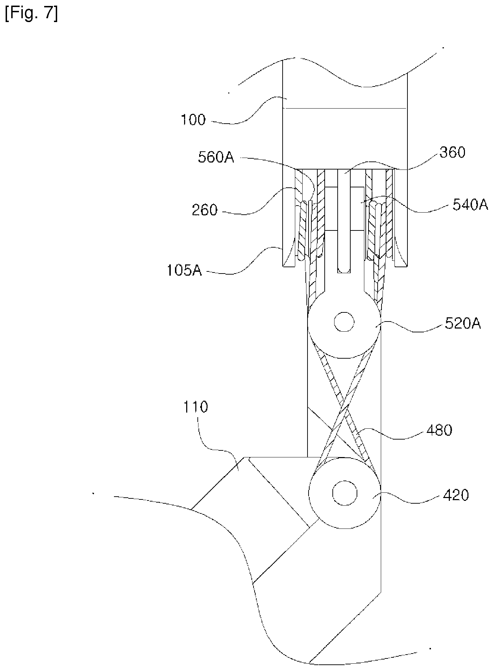

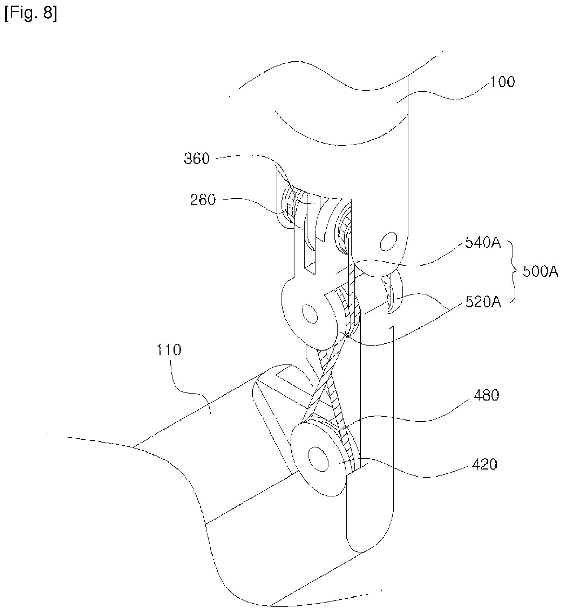

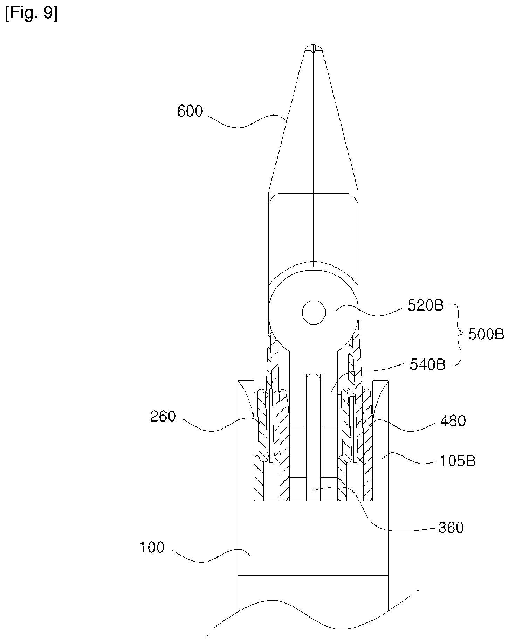

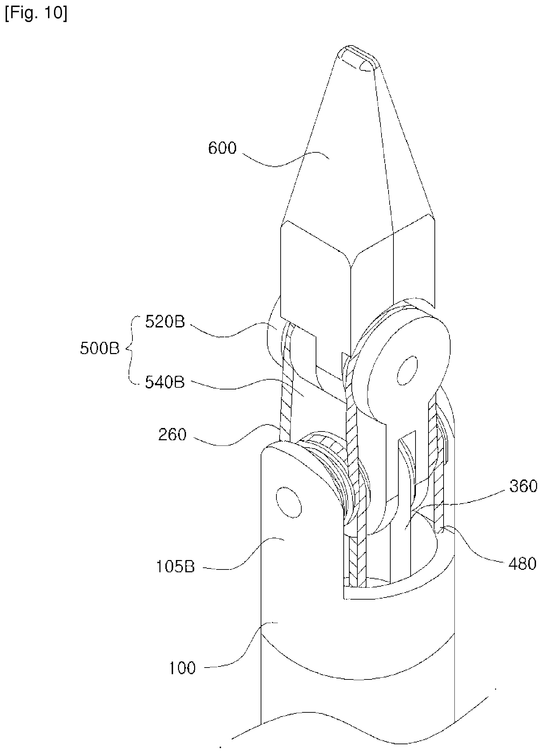

Next, the configuration of the pitch axis part 200 and the yaw axis part 300 which are involved in the rotation of the end effector 600 in a desired direction following the operation of the adjustment handle 110 will now be explained in further detail. As noted earlier, the pitch axis actuating cable and the yaw axis actuating cable are connected to the pitch axis part 200 and the yaw axis part 300, respectively, for transferring motions of the adjustment handle 110. Figs. 6, 7 and 8 show an installation status of cables on the side of the adjustment handle 110 in accordance with the first embodiment of the present invention, and Figs. 9, 10 and 11 show an installation status of cables on the side of the end effector 600 in accordance with the first embodiment of the present invention.

The previous drawings will also be referred in cooperation with Figs. 6, 7 and 8 and Figs. 9, 10 and 11 for the sake of more explicit explanations.

First, an example of how the yaw axis actuating cable 360 is connected will be described. The yaw axis actuating cable 360 winds around the first yaw axis cable pulley 320 and the second yaw axis cable pulley 340 that are disposed on either side of the elongated shaft 100, to thus transfer motions of the adjustment handle 110 in the yaw direction to the end effector 600. In accordance with a preferred embodiment of the present invention, although the yaw axis actuating cable 360 is wound around the first yaw axis cable pulley 320 and the second yaw axis cable pulley 340 to cause them to operate in the same direction, the yaw axis actuating cable 360 may be wound in a long '8' shape to cause the first yaw axis cable pulley 320 and the second yaw axis cable pulley 340 to operate in opposite directions, according to the user's needs. In either case, the yaw axis actuating cable 360 is wound through the shaft 100 having a space inside. Furthermore, if necessary, the first yaw axis cable pulley 320 and the second yaw axis cable pulley 340 may have different diameters from each other, such that the operation amount of the adjustment handle 110 and the displacement amount of the end effector 600 may be different from each other.

Even if the first yaw axis cable pulley 320 and the second yaw axis cable pulley 340 may have different diameters from each other, if the diameter ratio of the first yaw axis cable pulley 320 to the second yaw axis cable pulley 340 is not the same as the diameter ratio of the first connection pulleys 560A to the second connection pulleys 560B, the end effector 600 will not operate following the operation of the adjustment handle 110, thus causing deterioration in operational performance. Therefore, it is preferable to allow the end effector 600 to operate smoothly, by making the diameter ratio of the first yaw axis cable pulley 320 to the second yaw axis cable pulley 340 equal to the diameter ratio of the first connection pulleys 560A to the second connection pulleys 560B.

Meanwhile, as explained earlier, the first opening and closing cable pulley 420 is fixedly disposed on the rotation axis to which the first and the second rods 110A and 110B constituting the adjustment handle 110 are connected. Also, an opening and closing cable 480 is connected to the first opening and closing cable pulley 420 and to the second opening and closing cable pulley 440 which is disposed at a joint between the adjustment handle 110 and the first axis connection part 500A. In detail, the opening and closing cable 480 being connected to the second opening and closing cable pulley 440 winds around the second opening and closing cable pulley 440, as shown, and then it is connected to one out of the first connection pulley pair 560A on each side of the first axis connection part 500A. While being wound around the second opening and closing cable pulley 440, the opening and closing cable 480 is connected to the second axis connection part 500B, passing through the inner space of the shaft 100. Next, the opening and closing cable 480, while being wound around the first connection pulleys 560A, again winds around one out of the second connection pulley pair 560B on each side of the second axis connection part 500B, and then it also winds around the third opening and closing cable pulley 460 on the side of the end effector 600.

Referring to the drawings, the opening and closing cable 480 between the first and the second opening and closing cable pulleys 420 and 440 is preferably wound around in a long '8' shape. In accordance with another embodiment of the present invention, the first opening and closing cable pulley 420 may be formed on the first rod 110A of the adjustment handle 110, such that the opening and closing cable 480 may not need to be wound in crisscross form between the first and the second opening and closing cable pulleys 420 and 440. Also, if needed, the first opening and closing cable pulley 420 and the third opening and closing cable pulley 460 may have different diameters from each other, to thereby make the open/closed amount of the adjustment handle 110 and the open/closed amount of the end effector 600 different from each other.

In addition, the pitch axis actuating cable 260 is wound around the first pitch axis cable pulley 220 that is disposed on one end of the adjustment handle 110. The other end of the pitch axis actuating cable 260 winds around one particular connection pulley 560A without the opening and closing cable 480 being wound around it, which is selected out of the first connection pulleys 560A connected to the first yaw axis connection part 540A of the first axis connection part 500A, and it further winds around the second connection pulley 560B of the second axis connection part 500B that is connected to the other end of the shaft 100, passing through the inner space of the shaft 100.

Finally, the pitch axis actuating cable 260, which winds around the second connection pulley 560B, also winds around the second pitch axis cable pulley 240 disposed on one end of the end effector 600, to thereby transfer motions of the adjustment handle 110 in the pitch direction to the end effector 600.

Turning now to Fig. 9, the pitch axis actuating cable 260 winds around the farthest left pulley out of a pair of the second connection pulleys 560B on the left side of the second pitch axis connection part 520B, while the opening and closing cable 480 winds around the pulley next to it, such that two cables do not get entangled or rub against each other.

Under the connection by the pitch axis actuating cable 260 as above, the first pitch axis cable pulley 220 and the second pitch axis cable pulley 240 operate in the same direction. If necessary, however, the pitch axis actuating cable 260 may be wound in a way that the first and the second pitch axis cable pulleys 220 and 240 operate in opposite directions. In either case, the pitch axis actuating cable 260 is wound, passing through the inner space of the shaft 100. Here, according to the user s needs, the first pitch axis cable pulley 220 and the second pitch axis cable pulley 240 may be different in terms of diameter size, so as to make the operation amount of the adjustment handle 110 and the displacement amount of the end effector 600 different from each other. However, in order to prevent any deterioration in operational performance of the end effector 600, the diameter ratio of the first pitch axis cable pulley 220 to the second pitch axis cable pulley 240 is preferably the same as the diameter ratio of the second opening and closing cable pulley 440 to the third opening and closing cable pulley 460.

The following is an explanation about the operation of the tool 1 for minimally invasive surgery having the above-described configuration in accordance with the first embodiment of the present invention.

First, the tool 1 for minimally invasive surgery is aligned, as shown in Fig. 1.

A surgeon who performs the minimally invasive surgery puts his or her hand in the enclosure 112 of the adjustment handle 110 that is installed at one end of the tool 1 for minimally invasive surgery and holds the adjustment handle 110.

Hereinafter, it is assumed that (+) and (-) motions in the yaw direction designate motions in the right and left sides about the surgeon for convenience of explanation about the operation of the adjustment handle 110 in the yaw direction. Similarly, it is assumed that (+) and (-) motions in the pitch direction designate motions in the upper and lower sides about the surgeon for convenience of explanation about the operation of the adjustment handle 110 in the pitch direction.

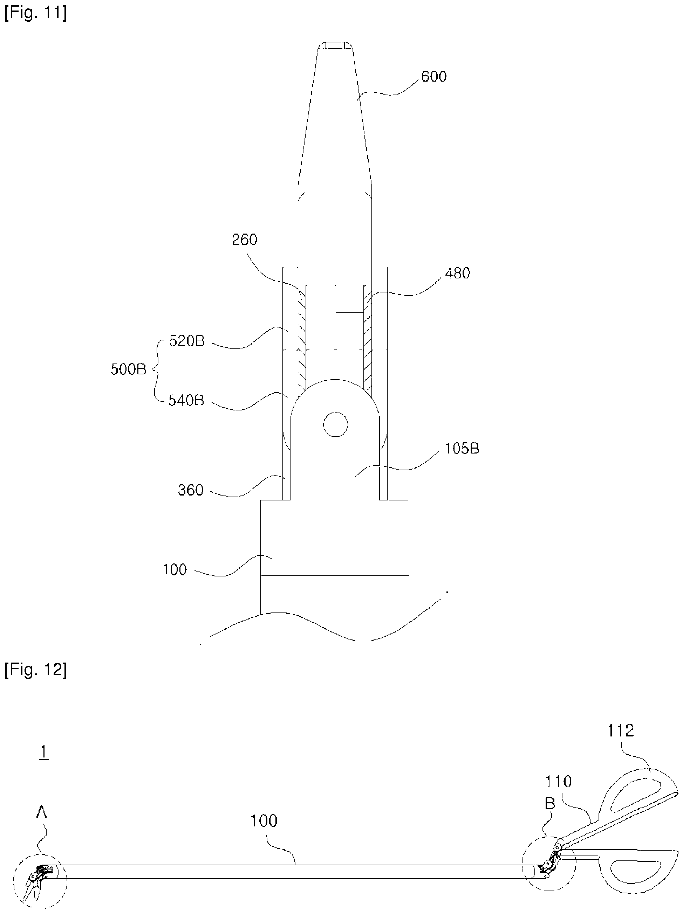

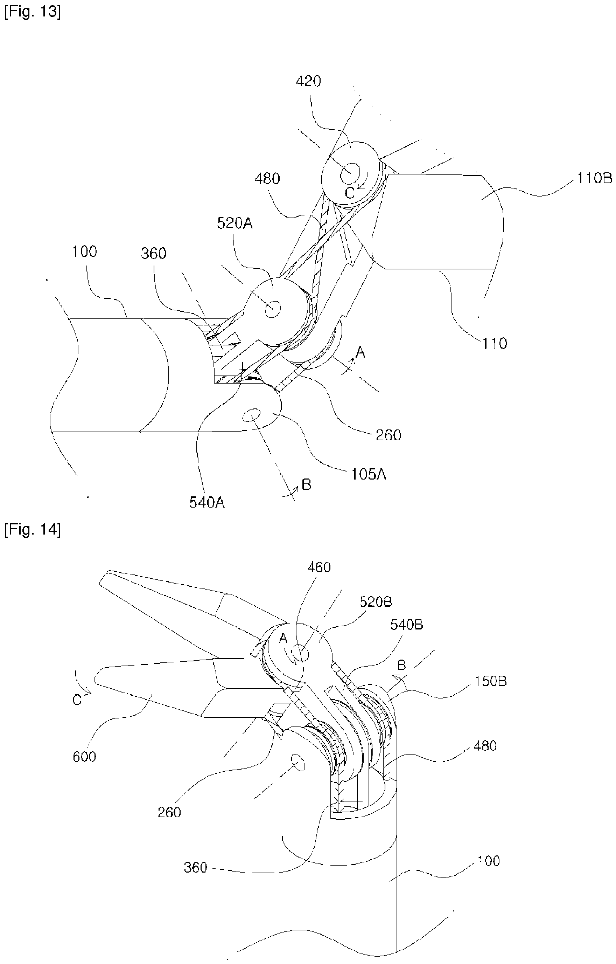

Fig. 12 illustrates a usage example of the tool 1 for minimally invasive surgery in accordance with the first embodiment of the present invention, Fig. 13 is a detailed view of 'B' portion in Fig. 12, and Fig. 14 is a detailed view of 'A' portion in Fig. 12.

When the surgeon, holing the adjustment handle 110 of the tool 1 for minimally invasive surgery being arranged as shown in Fig. 1, rotates the adjustment handle 110 upwardly (A direction) as depicted in Fig. 13, the first pitch axis cable pulley 220 and the second opening and closing cable pulley 440 that are disposed on one end of the adjustment handle 110 also rotate as much as a rotation amount of the adjustment handle 110. As such, when the first pitch axis cable pulley 220 and the second opening and closing cable pulley 440 rotate, the pitch axis actuating cable 260 and the opening and closing cable 480 which wind around the first pitch axis cable pulley 220 and the second opening and closing cable pulley 440, respectively, are pulled down on the lower side and released on the upper side. By this operation mechanism, the pitch axis actuating cable 260 and the opening and closing cable 480 drive (or rotate) the second pitch axis cable pulley 240 and the third opening and closing cable pulley 460. Accordingly, as shown in Figs. 12 and 14, the end effector 600 on which the second pitch axis cable pulley 240 and the third opening and closing cable pulley 460 are fixedly disposed face downwards.

Meanwhile, when the surgeon rotates the adjustment handle 110 to the left (B direction), the yaw axis actuating cable 360 wound around the first yaw axis cable pulley 320 and the second yaw axis cable pulley 340 is pulled on the right side and released on the left side. In addition, the pitch axis actuating cable 260 is pulled out upwards and downwards to the same degree as the first connection pulleys 560A connected to it rotates, while the opening and closing cable 480 is released both on the upper and bottom sides as the first connection pulleys 560A rotate. By this operation mechanism, the pitch axis actuating cable 260, the yaw axis actuating cable 360, and the opening and closing cable 480 drive (or rotate) the second axis connection part 500B in the yaw direction, and thus the end effector 600 faces the right direction, as shown in Fig. 14.

On the other hand, when the surgeon releases the adjustment handle 110 by opening up his hand, the adjustment handle 110 is opened while the second rod 110B rotates downwards (C direction). Following the opening of the adjustment handle 110, the first opening and closing cable pulley 420 rotates in a clockwise direction. Then, the rotation force produced by the clockwise rotation of the first opening and closing cable pulley 420 drives (or rotates) the second opening and closing cable pulley 440 by means of the opening and closing cable 480. At this time, the first opening and closing cable 420 and the second opening and closing cable pulley 440 rotate in opposite directions from each other by the opening and closing cable 480. Here, the motion of the second opening and closing cable pulley 440 is transferred to the third opening and closing cable pulley 460 and further to the lower rod of the end effector 600 to which the third opening and closing cable pulley 460 is secured, thereby opening the end effector 600 in the direction C.

The operation set forth above has been made in order of the motion in the pitch direction, the motion in the yaw direction, and the opening motion of the end effector 600 to get a better understanding on each motion, but the motions may not follow the exact order described here. Instead, two or more of them may be carried out concurrently. Even so, the same operation results can be obtained based on the same operational principle as described earlier.

Meanwhile, when the motion of the adjustment handle 110 is transferred to the end effector 600 as noted above, the transfer of the motion may not be done smoothly as expected, depending on which direction the end effector 600 has rotated. For instance, suppose that the surgeon rotated the adjustment handle 110 in the yaw direction. In this case, although the first and the second yaw axis cable pulleys 320 and 340 that are connected in parallel in vicinity of the shaft 100 can facilitate the transfer of motions in the yaw direction, the motion in the pitch direction or the opening and closing operations of the adjustment handle 110 may not be always smoothly transferred to the end effector 600.

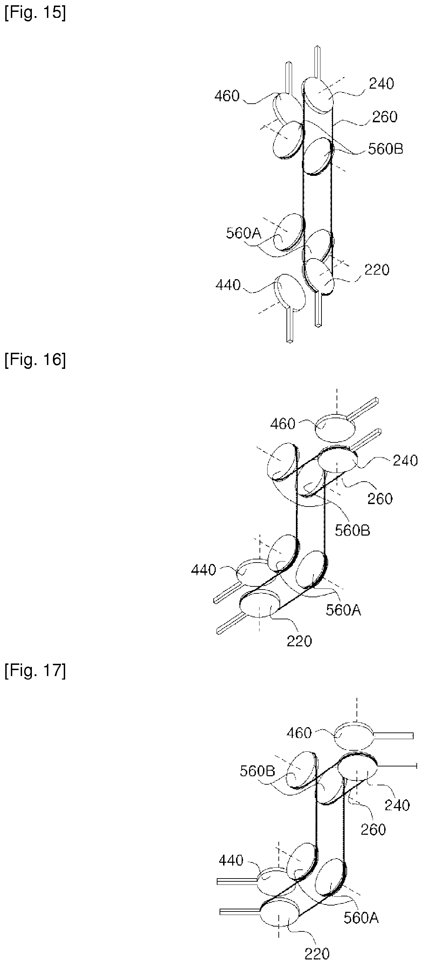

Therefore, in order to facilitate the transfer of the motion in the pitch direction and the opening and closing operations of the adjustment handle 110 to the end effector 600, first and second connection pulleys 560A and 560B similar to those explained before are additionally provided to the first and the second axis connection parts 500A and 500B. Further details about configurations and functions of the first and the second connection pulleys 560A and 560B will be provided below, with reference to Figs. 15, 16 and 17.

Figs. 15, 16 and 17 are conceptual diagrams describing the principle of motion transfer of the first and the second connection pulleys 560A and 560B in accordance with the first embodiment of the present invention. As depicted in Figs. 15, 16 and 17, by the use of the first and the second connection pulleys 560A and 560B in accordance with the first embodiment of the present invention, although the end effector 600 may have been rotated in the yaw direction, the transfer of motion by the pitch axis actuating cable 260 becomes easier by the first and the second connection pulleys 560A and 560B. Needless to say, the transfer of motion by the cable 480 becomes also easier, based on the same principle.

First, Fig. 15 shows a status where the adjustment handle 110 is aligned with the end effector 600, the pitch axis actuating cable 260 is wound around the first and the second pitch axis cable pulleys 220 and 240, and the first and the second connection pulleys 560A and 560B are interposed therebetween.

Fig. 16 shows a status where the adjustment handle 110 and the end effector 600 rotated to a certain degree in the yaw direction. In this case, the rotation of the end effector 600 in the yaw direction corresponding to the rotation of the adjustment handle 110 in the yaw direction may occur by the yaw axis actuating cable 360 (not shown in Fig. 16), but it can also occur by the operation of the first pitch axis cable pulley 220 and of the second opening and closing cable pulley 440. In this case, the first and the second connection pulleys 560A and 560B are responsible for the transfer of the operation of the first pitch axis cable pulley 220 and of the second opening and closing cable pulley 440 in the yaw direction to the second pitch axis cable pulley 240 and the third opening and closing cable pulley 460.

Fig. 17 shows a status where the adjustment handle 110 that was in the status shown in Fig. 16 is not operated in the pitch direction. As depicted in the drawing, the first and the second connection pulleys 560A and 560B are responsible for the transfer of the operation of the first pitch axis cable pulley 220 and of the second opening and closing cable pulley 440 in the pitch direction to the second pitch axis cable pulley 240 and the third opening and closing cable pulley 460.

To make it sure that the functions of the first and the second connection pulleys 560A and 560B set forth above can be smoothly realized, the first pitch axis cable pulley 220 and the second opening and closing cable pulley 440 are preferably arranged in such a manner that their centers are located at the same distance from the first connection pulley pair 560A. Similarly, the second pitch axis cable pulley 240 and the third opening and closing cable pulley 460 are also preferably arranged in such a manner that their centers are located at the same distance from the second connection pulley pair 560B. Moreover, a size gap between the first pitch axis cable pulley 220 and the second opening and closing cable pulley 440 is the same as the diameter of the first connection pulleys 560A, and a size gap between the second pitch axis cable pulley 240 and the third opening and closing cable pulley 460 is the same as the diameter of the second connection pulleys 560B.

### Aspect II

Unlike the first embodiment of the present invention discussed above which suggested the tool 1 for minimally invasive surgery utilizing the opening and closing end effector 600, a tool for minimally invasive surgery which does not always absolutely require the opening and closing operation may be addressed. For instance, a hook electrode used for the end effector may be addressed.

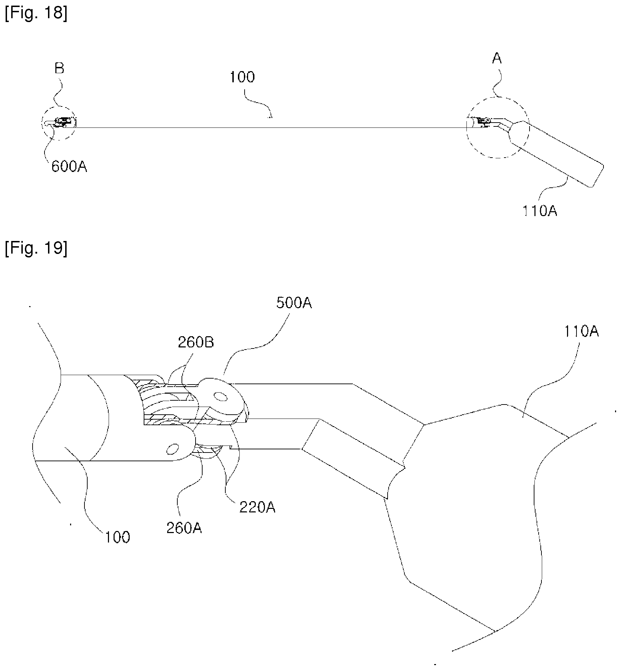

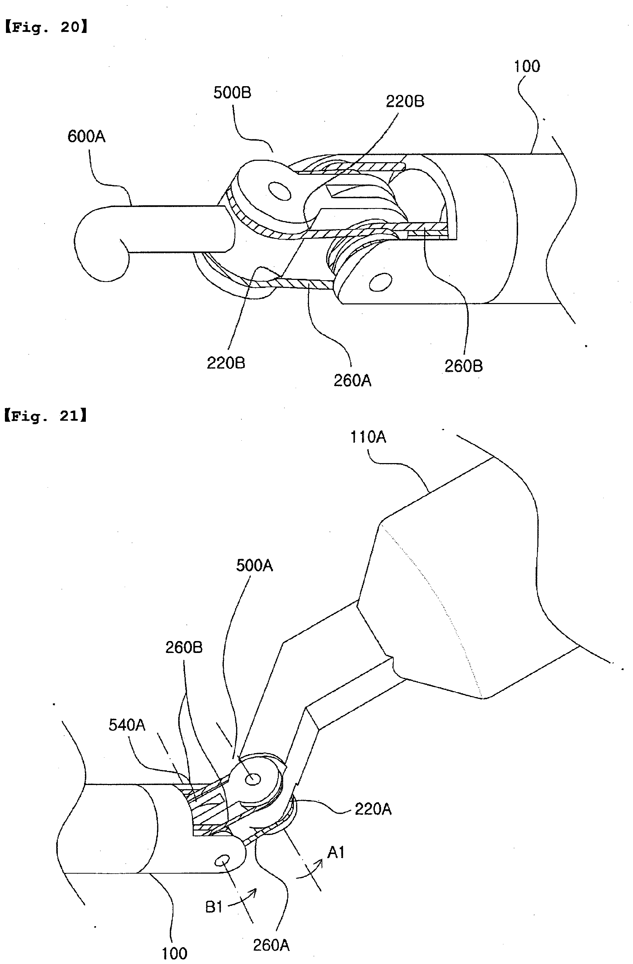

Fig. 18 is a perspective view showing the outer appearance of a tool for minimally invasive surgery in accordance with a second aspect of the present disclosure, and Figs. 19 and 20 show a detailed view of "A' part and 'B' part in Fig. 18.

Referring to Figs. 18 to 20, an adjustment handle 110A for controlling the operation of an end effector 600A in hook-electrode form is connected to one end of an elongated shaft 100 by a first axis connection part 500A, and the end effector 600A is connected to the other end of the elongated shaft 100 by a second axis connection part 500B. Unlike the first embodiment, the end effector 600A of this aspect may take the form of a bar (or any other form such as a ring shape as long as the opening and closing operation is not accompanied).

The connections of the first and the second axis connection parts 500A and 500B and the cables are done basically similar to those in the first embodiment except that the yaw axis actuating cable(s) and the opening and closing cable(s) are not required. Therefore, only a brief description thereof will be provided below.

A pair of first cable pulleys 220A is formed on both sides of a joint between the adjustment handle 110A and the first axis connection part 500A such that they interwork with the operation of the adjustment handle 110A. Also, a first cable 260A and a second cable 260B wind around the pair of the first cable pulleys 220A, respectively.

The end effector 600A is disposed on the other end of the elongated shaft 100 with the second axis connection part 500B interposed between them. A pair of second cable pulleys 220B which are wound with the first and the second cables 260A and 260B, respectively, is disposed on both sides of a rotation axis connecting the second axis connection part 500B with the end effector 600A, thereby transferring the operation of the adjustment handle 110A to the end effector 600A. Also, the configuration shown in Figs. 15 to 17 in relation to the first embodiment (namely, the configuration using the first and the second connection pulleys 560A and 560B) can equally be adapted to this aspect.

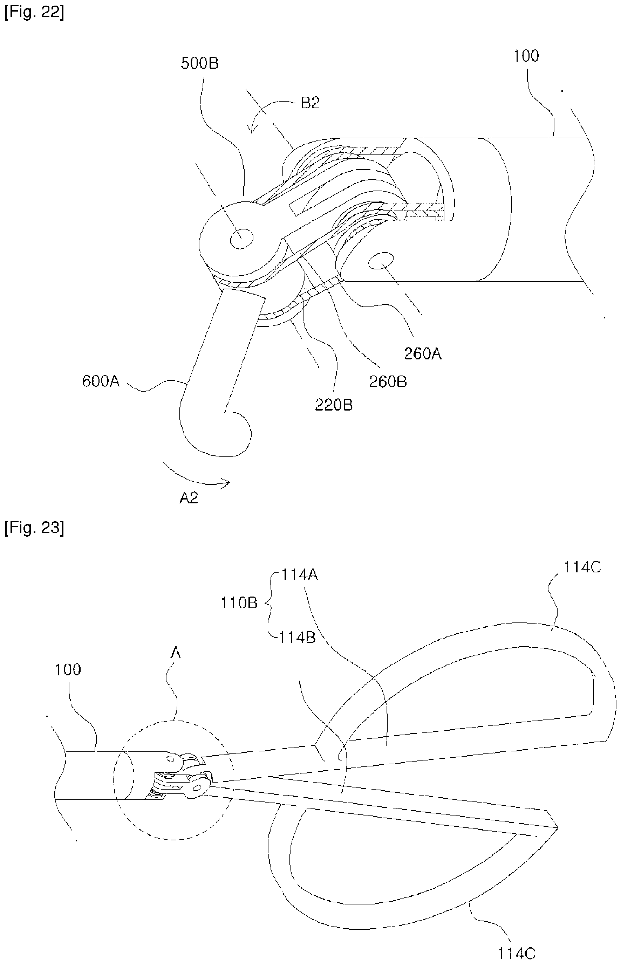

Figs. 21 and 22 show a usage example of the tool for minimally invasive surgery in accordance with the second aspect of the present disclosure, which is not part of the present invention. With reference to Figs. 21 and 22, the operation of the tool for minimally invasive surgery will be described below.

When a surgeon holding the adjustment handle 110A rotates it in the counterclockwise direction of a pitch axis (A1 direction), the first and the second cables 260A and 260B connected to the first cable pulley 220A are pulled on the lower side and released on the upper side, and thus the motion of the adjustment handle 110A in the pitch direction is transferred to the end effector 600A. At this time, the second cable pulleys 220B disposed on both sides of the rotation axis of the end effector 600A rotate in the counterclockwise direction (A2 direction) by the first and the second cables 260A and 260B.

Again, when the surgeon rotates the adjustment handle 110A to the left of the yaw axis (B1 direction), the first cable 260A wound around the connection pulley on one side of the first yaw axis connection part 540A of the first axis connection part 500A is pulled out, while the second cable 260B wound around another connection pulley on the other side of the first yaw axis connection part 540A is released. In result, the motion of the adjustment handle 110A in the yaw direction is transferred to the end effector 600A, thereby causing the end effector 600A to rotate to the right of the yaw axis (B2 direction).

### Embodiment III

Referring next to Figs. 23 to 26, a third embodiment of the present invention in which an end effector 600B operates as two rods 114A and 114B that constitute an adjustment handle 110B operate concurrently will be discussed.

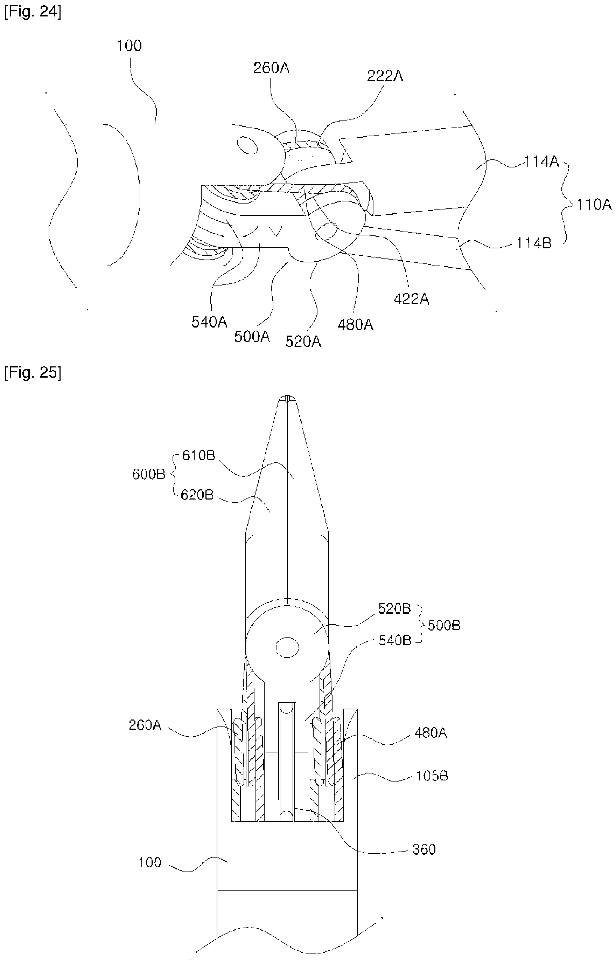

Fig. 23 is a perspective view showing the configuration of the adjustment handle 110B used for a tool for minimally invasive surgery in accordance with the third embodiment of the present invention, and Fig. 24 is a detailed view of A part in Fig. 23.

To configure the adjustment handle 110B disposed on one end of the elongated shaft 100, one ends of the first and the second rods 114A and 114B are connected to each other by a rotation axis, and two enclosures 114C of a semi-circular shape are formed symmetrically to each other on one side of each of the first and the second rods 110A and 110B. Also, first and second cable pulleys 222A and 422A are positioned on either side of the rotation axis that connects the first rod 114A with the second rod 114B. Here, the first and the second cable pulleys 222A and 422B are configured to interwork with the operations of the second and the first rods 114B and 114A, respectively, and a first cable 260A and a second cable 480A are connected to the first cable pulley 222A and the second cable pulley 422A, respectively. Besides, the first and the second cable pulleys 222A and 422A are positioned on the inside of a first pitch axis connection part 520A of a first axis connection part 500A in a rotatable manner. The first cable 260A and the second cable 480A each wind around the connection pulleys that are formed on both sides of a first yaw axis connection part 540A.

A second axis connection part 500B is formed on the other end of the elongated shaft 100, and cable pulleys that correspond to the first and the second cables pulleys 222A and 422A are settled on both sides of the second pitch axis connection part 520B of the second axis connection part 500B. In this way, power being transferred through the first and THE second cables 260A and 480A is used to control the rotation of the rods 620B and 610B which constitute the end effector 600B. Again, the configuration shown in Figs. 15 to 17 in relation to the first embodiment (namely, the configuration using the first and the second connection pulleys 560A and 560B) can equally be applied to this embodiment.

Similar to the tool for minimally invasive surgery described in each of the previous embodiments, the operation of the tool for minimally invasive surgery in accordance with this embodiment is carried out in a manner that operations in the pitch and yaw directions are controlled by means of the two cables 260A and 480A, and the end effector 600B may be opened or closed especially when those two cables 260A and 480A have different displacement amounts from each other in the pitch direction (i.e., either they operate in opposite directions or they operate in the same direction with different displacement amounts). That is, according to the drawings, the end effector 600B can be opened when the first rod 114A and the second rod 114B are separated from each other. Needless to say, it may work the other way around. That is, depending on how the cables 260A and 480A are configured, the end effector 600B may be closed when the first rod 114A and the second rod 114B are separated from each other.

### Embodiment IV

This embodiment introduces a more simplified configuration using fewer cables for the operation of an end effector of a tool for minimally invasive surgery.

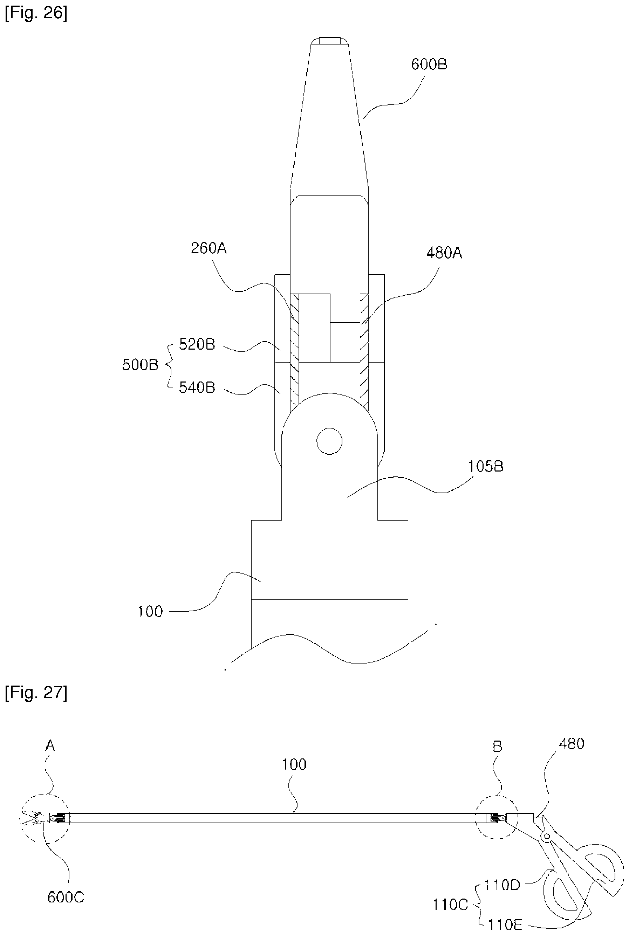

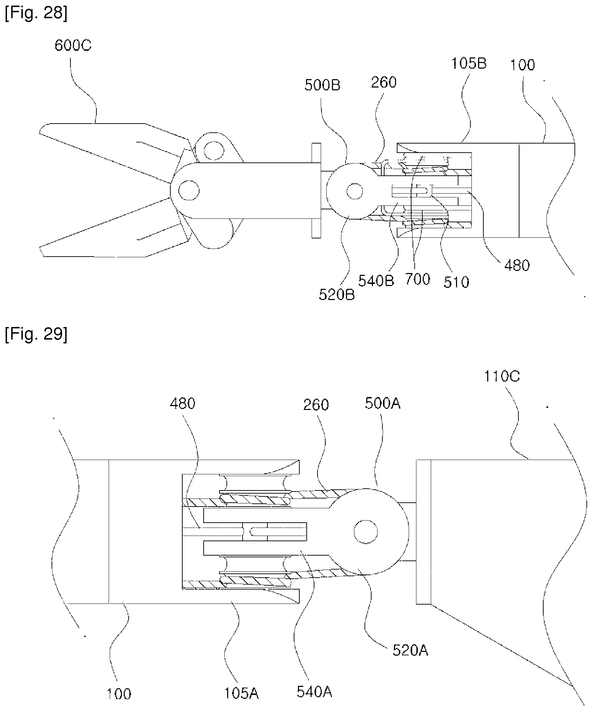

Fig. 27 shows the configuration of a tool for minimally invasive surgery in accordance with a fourth embodiment of the present invention, and Figs. 28 and 29 show a detailed view of 'A' part and 'B' part in Fig. 27.

As shown in the drawings, the fourth embodiment of the present invention also uses pitch axis actuating cables 260 as well as opening and closing cables 480, as in the first embodiment. In addition, a restoration spring 700 winds around a connection pulley on each end of a second yaw axis connection part 540B of a second axis connection part 500B, thereby applying a predetermined level of elastic force for the operation in the yaw direction. Here, the opening and closing cable 480 is connected to an end effector 600C via a through hole 512 on the central axis of the second axis connection part 500B.

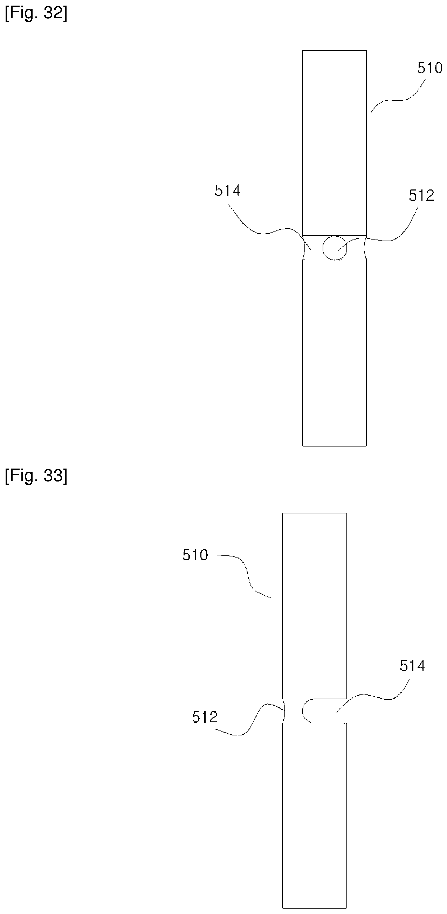

When an axis 510 rotates with the opening and closing cable 480 being inserted into the through hole 512, the opening and closing cable 480 is pulled out and the end effector 600C may be opened or closed, contradicting the intention of a surgeon. To prevent this, as shown in Figs. 32 and 33, a cable groove 514 into which the opening and closing cable 480 is inserted is formed around the through hole 512. By doing so, even if the axis 510 may rotate, no unintended operation (i.e., opening or closing) of the end effector 600C will take place. Here, the depth of the cable groove 514 preferably exceeds a half of the diameter of the axis 510. Moreover, as shown in the drawings, such a cable groove may be formed in the central axis of a first pitch axis connection part 520A, the central axis of a second pitch axis connection part 520B, the central axis of a first yaw axis connection part 540A, or the central axes of some designated rotational elements, if necessary.

Further details on the configuration and operational principle of the opening and closing cable according to this embodiment are provided in Korean Patent Application No. 2008-51248 as a related application, filed by the same applicant.

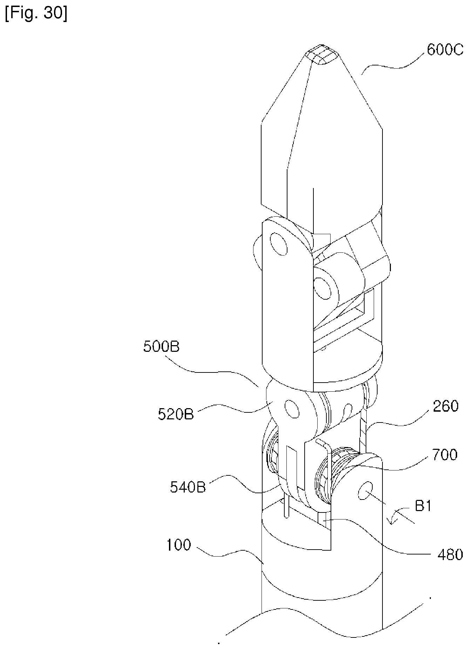

In accordance with this embodiment, the end effector 600C may operate in the pitch direction by the operation of the pitch axis actuating cable 260 as in the first embodiment, or may be opened or closed by the operation of the opening and closing cable 480 as explained in the related application. Meanwhile, using the connection pulleys described in Figs. 15, 16 and 17 may also control the operation of the end effector 600C in the yaw direction, in cooperation with the pitch axis actuating cable 260. In this case, a restoration spring 700 can be used to apply an elastic force for deflecting the end effector 600C in normal state to a yaw direction (e.g., direction B1 in Fig. 30). As a result, it may become easier to control the operation of the end effector 600C in the yaw direction with the help of the pitch axis actuating cable 260. In other words, for instance, if the end effector 600C needs to operate in the opposite direction to the direction B1 of the yaw axis, the pitch axis actuating cable 260 is involved to make the corresponding operation in the yaw direction occur. Meanwhile, if the end effector 600C needs to operate in the direction B1 of the yaw axis, stress on the pitch axis actuating cable 260 is relieved to get more dependent on the restoring force of the restoration spring 700.

Referring to Fig. 28 in relation to this embodiment, the restoration spring 700 is disposed on both sides of the yaw axis connection part 540B of the second axis connection part 500B, but the present invention is not limited to such configuration. That is to say, it is obvious to those skilled in the art to which the present invention pertains to that the restoration spring 700, depending on its elastic force, may be disposed only on one side of the yaw axis connection part 540B or in any other position to restore the operation of the end effector 600C in the yaw direction.

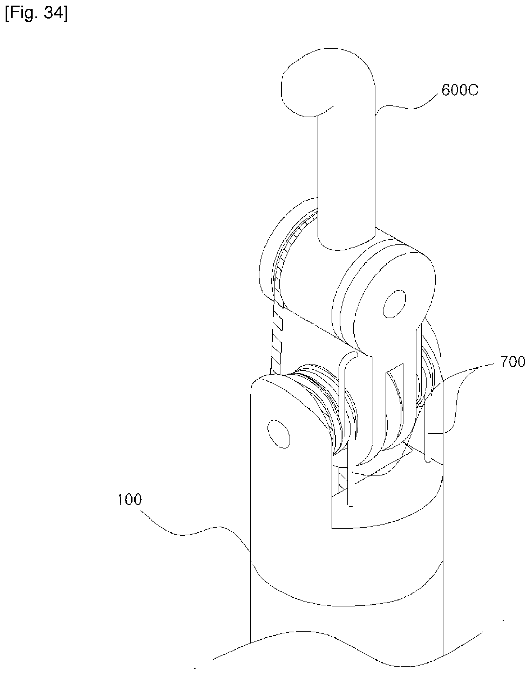

Meanwhile, as shown in Fig. 34, when an end effector 600C is configured with an element, such as a hook electrode, that does not perform the opening and closing operation at all, opening and closing cables are no longer required to devise a tool for minimally invasive surgery, but only the pitch axis actuating cables and the restoration spring 700 are sufficient to enable all operations in the pitch and yaw directions.

While the present invention has been described with respect to certain preferred embodiments, it will be apparent to those skilled in the art that various changes and modifications may be made without departing from the scope of the invention as defined in the following claims.

## Claims

1. Tool for minimally invasive surgery comprising:
an elongated shaft (100) having a predetermined length;
an adjustment handle (110) manually controllable by a user,
a first pitch axis part and a first yaw axis part positioned around one end of the elongated shaft (100) for transferring motions in pitch and yaw directions following the operation of the adjustment handle (110);
a second pitch axis part and a second yaw axis part positioned around the other end of the elongated shaft (100) for operating corresponding to the operations from the first pitch axis part and the first yaw axis part, respectively,
an end effector (600) controllable by the second pitch axis part and the second yaw axis part;
a pair of connection pulleys (560A, 560B); and
a first axis connection part (500A) connecting the adjustment handle (110, 110A) with one end of the elongated shaft (100) and a second axis connection part (500B) connecting the other end of the elongated shaft (100) with the end effector (600),
wherein the first pitch axis part and the first yaw axis part drive the second pitch axis part and the second yaw axis part by at least one actuating cable (260A, 260B), respectively, and
wherein the pair of connection pulleys (560A, 560B) transfers an operation of the first pitch axis part in the yaw direction to the second pitch axis part,
wherein the at least one actuating cable (260A, 260B) comprises at least one first cable (260A) and at least one second cable (260B), and the first pitch axis part and the first yaw axis part drive the second pitch axis part and the second yaw axis part by the at least one first cable (260A) and the at least one second cable (260B),
wherein the end effector (600) is configured to be opened or closed by the second pitch axis part and the second yaw axis part according to the difference in the displacement amount of the at least one first cable and the at least one second cable,
**characterized in that** at least one of the first axis connection part (500A) and the second axis connection part (500B) comprises:
a pitch axis connection part (520A, 520B) having a pair of plates spaced apart from each other; and
a yaw axis connection part (540A, 540B) having a pair of plates spaced apart from each other, and connected to either the first yaw axis part or to the second yaw axis part,
wherein the pitch axis connection part (520A, 520B) and the yaw axis connection part (540A, 540B) are coupled orthogonally, and
wherein the pitch axis connection part (520A) is connected to the first pitch axis part when the yaw axis connection part (540A) is connected to the first yaw axis part, and the pitch axis connection part (520B) is connected to the second pitch axis part when the yaw axis connection part (540B) is connected to the second yaw axis part.

2. The tool as claimed in claim 1, wherein the first yaw axis part further drives the second yaw axis part by at least one restoration spring (700).

## Patentansprüche

1. Instrument für minimalinvasive Chirurgie umfassend:
einen langgestreckten Schaft (100), der eine vorgegebene Länge aufweist;
einen Einstellungsgriff (110), der von einem Benutzer manuell steuerbar ist;
ein erstes Nickachsenteil und ein erstes Gierachsenteil, die um ein Ende des langgestreckten Schafts (100) herum positioniert sind, um nach der Betätigung des Einstellungsgriffs (110) Bewegungen in Nick- und Gierrichtungen zu übertragen;
ein zweites Nickachsenteil und ein zweites Gierachsenteil, die um das andere Ende des langgestreckten Schafts (100) herum positioniert sind, um entsprechend den Betätigungen von dem ersten Nickachsenteil bzw. dem ersten Gierachsenteil zu arbeiten;
einen Endeffektor (600), der durch das zweite Nickachsenteil und das zweite Gierachsenteil steuerbar ist;
ein Paar von Verbindungsumlenkrollen (560A, 560B); und
ein erstes Achsenverbindungsteil (500A), das den Einstellungsgriff (110, 110A) mit einem Ende des langgestreckten Schafts (100) verbindet, und ein zweites Achsenverbindungsteil (500B), das das andere Ende des langgestreckten Schafts (100) mit dem Endeffektor (600) verbindet,
wobei das erste Nickachsenteil und das erste Gierachsenteil das zweite Nickachsenteil und das zweite Gierachsenteil jeweils durch wenigstens ein Betätigungskabel (260A, 260B) antreiben, und
wobei das Paar von Verbindungsumlenkrollen (560A, 560B) eine Betätigung des ersten Nickachsenteils in der Gierrichtung zu dem zweiten Nickachsenteil überträgt,
wobei das wenigstens eine Betätigungskabel (260A, 260B) wenigstens ein erstes Kabel (260A) und wenigstens ein zweites Kabel (260B) umfasst, und das erste Nickachsenteil und das erste Gierachsenteil das zweite Nickachsenteil und das zweite Gierachsenteil durch das wenigstens eine erste Kabel (260A) und das wenigstens eine zweite Kabel (260B) antreiben,
wobei der Endeffektor (600) dazu konfiguriert ist, durch das zweite Nickachsenteil und das zweite Gierachsenteil gemäß dem Unterschied des Verschiebungsbetrags des wenigstens einen ersten Kabels und des wenigstens einen zweiten Kabels geöffnet oder geschlossen zu werden,
**dadurch gekennzeichnet, dass** wenigstens eines des ersten Achsenverbindungsteils (500A) und des zweiten Achsenverbindungsteils (500B) umfasst:
ein Nickachsenverbindungsteil (520A, 520B), das ein Paar von Platten aufweist, die voneinander beabstandet sind; und
ein Gierachsenverbindungsteil (540A, 540B), das ein Paar von Platten aufweist, die voneinander beabstandet sind, und mit entweder dem ersten Gierachsenteil oder dem zweiten Gierachsenteil verbunden sind,
wobei das Nickachsenverbindungsteil (520A, 520B) und das Gierachsenverbindungsteil (540A, 540B) orthogonal gekoppelt sind, und
wobei das Nickachsenverbindungsteil (520A) mit dem ersten Nickachsenteil verbunden ist, wenn das Gierachsenverbindungsteil (540A) mit dem ersten Gierachsenteil verbunden ist, und das Nickachsenverbindungsteil (520B) mit dem zweiten Nickachsenteil verbunden ist, wenn das Gierachsenverbindungsteil (540B) mit dem zweiten Gierachsenteil verbunden ist.

2. Instrument nach Anspruch 1, wobei das erste Gierachsenteil ferner das zweite Gierachsenteil durch wenigstens eine Rückstellfeder (700) antreibt.

## Revendications

1. Outil pour chirurgie minimalement invasive comprenant :
- une tige allongée (100) présentant une longueur prédéterminée,
- une poignée d'ajustement (110) contrôlable manuellement par un utilisateur,
- une première pièce d'axe de tangage et une première pièce d'axe de lacet placées autour d'une extrémité de la tige allongée (100) pour transférer les mouvements dans les directions de tangage et de lacet à la suite de l'opération de la poignée d'ajustement (110),
- une deuxième pièce d'axe de tangage et une deuxième pièce d'axe de lacet placées autour de l'autre extrémité de la tige allongée (100) pour une opération correspondant aux opérations provenant de la première pièce d'axe de tangage et de la première pièce d'axe de lacet, respectivement,
- un effecteur terminal (600) contrôlable par la deuxième pièce d'axe de tangage et la deuxième pièce d'axe de lacet,
- une paire de poulies de connexion (560A, 560B), et
- une première pièce de connexion axiale (500A) connectant la poignée d'ajustement (110, 110A) avec une extrémité de la tige allongée (100) et une deuxième pièce de connexion axiale (500B) connectant l'autre extrémité de la tige allongée (100) avec l'effecteur terminal (600),
- où la première pièce d'axe de tangage et la première pièce d'axe de lacet entraînent la deuxième pièce d'axe de tangage et la deuxième pièce d'axe de lacet par l'intermédiaire d'au moins un câble d'actionnement (260A, 260B), respectivement, et
- où la paire de poulies de connexion (560A, 560B) transfère une opération de la première pièce d'axe de tangage dans la direction de lacet à la deuxième pièce d'axe de tangage,
- où le au moins un câble d'actionnement (260A, 260B) comprend au moins un premier câble (260A) et au moins un deuxième câble (260B), et la première pièce d'axe de tangage et la première pièce d'axe de lacet entraînent la deuxième pièce d'axe de tangage et la deuxième pièce d'axe de lacet par l'intermédiaire du au moins un premier câble (260A) et du au moins un deuxième câble (260B),
- où l'effecteur terminal (600) est configuré pour être ouvert ou fermé par la deuxième pièce d'axe de tangage et la deuxième pièce d'axe de lacet en fonction de la différence dans la quantité de déplacement entre le au moins un premier câble et le au moins un deuxième câble,
- **caractérisé en ce qu'**au moins une parmi la première pièce de connexion axiale (500A) et la deuxième pièce de connexion axiale (500B) comprend :
- une pièce de connexion de l'axe de tangage (520A, 520B) présentant une paire de plaques séparées l'une de l'autre, et
- une pièce de connexion de l'axe de lacet (540A, 540B) présentant une paire de plaques séparées l'une de l'autre,
- et connectées soit à la première pièce d'axe de lacet soit à la deuxième pièce d'axe de lacet,
- où la pièce de connexion de l'axe de tangage (520A, 520B) et la pièce de connexion de l'axe de lacet (540A, 540B) sont couplées orthogonalement, et
- où la pièce de connexion de l'axe de tangage (520A) est connectée à la première pièce de l'axe de tangage quand la pièce de connexion de l'axe de lacet (540A) est connectée à la première pièce de l'axe de lacet, et la pièce de connexion de l'axe de tangage (520B) est connectée à la deuxième pièce de l'axe de tangage quand la pièce de connexion de l'axe de lacet (540B) est connectée à la deuxième pièce de l'axe de lacet.

2. Outil selon la revendication 1, **caractérisé en ce que** la première pièce de l'axe de lacet entraîne en outre la deuxième pièce de l'axe de lacet par au moins un ressort de restauration (700).
